# EUROPEAN PATENT SPECIFICATION

(11) **EP 2 395 927 B1**
(45) Date of publication and mention of the grant of the patent: **19.10.2016**
(21) Application number: 09779036.4
(22) Date of filing: 10.02.2009
(51) Int. Cl.: A61B 17/072, A61B 17/00, A61B 17/29, A61B 90/00

(54) **AN INDICATOR DEVICE FOR INDICATING PROPERTIES OF BODY TISSUE SUBJECTED TO SURGICAL STAPLING AS A FUNCTION OF TISSUE COMPRESSION TIME**
INDIKATORVORRICHTUNG ZUR ANZEIGE DER EIGENSCHAFTEN VON KÖRPERGEWEBE, DAS CHIRURGISCH GEKLAMMERT WIRD, ALS FUNKTION DER GEWEBEKOMPRESSIONSZEIT
DISPOSITIF INDICATEUR DESTINÉ À INDIQUER LES PROPRIÉTÉS D'UN TISSU CORPOREL SOUMIS À UN AGRAFAGE CHIRURGICAL EN FONCTION D'UN TEMPS DE COMPRESSION DU TISSU

(43) Date of publication of application: 21.12.2011
(73) Proprietor: Ethicon Endo-Surgery, Inc., Cincinnati, OH 45242 (US)
(72) Inventor: BAZZURRI, Fabrizio, I-00041 Albano Laziale Roma (IT); D'ARCANGELO, Michele, I-00142 Roma (IT); BILOTTI, Federico, I-04011 Aprilia, Latina (IT); BALEK, Stephen, I-00124 Casal Palocco, Roma (IT)
(74) Representative: Leihkauf, Steffen Falk
(86) International application number: PCT/EP2009/051513
(87) International publication number: WO 2010/091718

(56) References cited:
- EP-A- 1 813 199
- WO-A-2007/121579
- US-A1- 2006 278 680

## Description

The invention relates in general to the field of surgical stapling of body tissue by means of surgical staplers and in particular to an indicator device for indicating properties of body tissue subjected to surgical stapling as a function of tissue compression time.

Known surgical staplers comprise an insertion shaft, a handle portion fitted to a distal end of the insertion shaft and a tissue manipulation portion fitted to a proximal end of the insertion shaft. The tissue manipulation portion holds a cartridge device which houses a group of surgical staplers and an anvil having a staple forming surface suitable to co-operate with an end surface of the cartridge device in order to clamp layers of tissue therebetween and to bend the ends of the staples when they are expelled from the cartridge device against the anvil.

The known surgical staplers further comprise an anvil approximation device or, with other words, a staple jaw approximation device and a staple driving device.

The anvil approximation device includes a mechanism which is configured to move the anvil towards the cartridge device in order to clamp the tissue prior to and during the application of the staples as well as to widen the distance between the anvil and the cartridge device after the stapler has been "fired" in order to release the stapled tissue.

In so called circular staplers, the anvil approximation device is often operated by a rotating knob arranged at the distal end of the handle portion and provided with a movement transmission and transformation mechanism which is functionally connected to both the rotating knob and the tissue manipulating device in order to allow to adjust the effective distance (so called "staple height") between the proximal end surface of the cartridge device and the staple forming surface of the anvil.

In so called linear staplers or endocutters, the anvil approximation device is often operated by an approximating lever arranged at the handle portion and provided with a movement transmission and transformation mechanism which is functionally connected to both the approximating lever and the tissue manipulating device in order to allow to adjust the effective distance between the end surface of the cartridge device jaw and the staple forming surface of the anvil jaw.

The staple driving device comprises an operating member arranged at the handle portion of the stapler and a mechanism which transforms the movement of the operating member (e.g. a trigger lever) in a movement of a staple driving platform inside the cartridge device which drives ("fires") the surgical staples out of the cartridge device, across the clamped tissue layers and against the staple forming surface of the anvil so that the staple ends are bent in a way to give the staple a more or less C or B shaped configuration.

In known surgical linear staplers or endocutters, e.g. endocutters EZ®, ETS® and Echelon® by Ethicon Endo-Surgery, Inc., once the cartridge device jaw and the anvil jaw are closed through activation of the approximating lever, they apply a substantially constant compression force to the tissue layers clamped therebetween, thereby performing what is commonly known as "wide compression" of the tissue.

For human body tissue being composed of both fluid and solid, it evacuates part of its fluids when subjected to "wide compression" and its volume and thickness decrease till, after a few seconds of compression, it reaches a correct thickness and residual fluid content for an optimal staple formation.

In case the surgeon does not wait a minimum period of compression time between activation of the anvil approximation mechanism and the subsequent activation of the staple drive mechanism, the stapled tissue layers will not yet have achieved the correct thickness and consistency and staple formation will be not optimal and, in some cases, an unnecessarily large staple height and staple type might be required.

Experimental tests have shown that, under normal endocutter anvil jaw compression, human tissue layer thickness decreases by about 0.5 mm over a period of time of 15 seconds, which is a minimum value of compression time suggested e.g. for the use of the above cited known surgical staplers. Known surgical staplers with compression time display are described in US2006/0278680. However the known staplers don't indicate the tissue thickness reduction for a selected compression time value and are therefore not suitable for a thorough understanding of the effect of wide compression.

US 2006/0278680 discloses a surgical apparatus having a clamp and a stapling mechanism. The clamp has a first jaw and a second jaw to clamp on a body tissue at a desired location for a stapling operation. The surgical apparatus has a controller for providing a delay between clamping and actuating of the firing mechanism of the stapling mechanism.

Even though the described behavior of body tissue subjected to wide compression is generally well understood from a theoretical viewpoint, often it is not duly taken into account by surgeons during tissue suturing and resection by means of surgical stapling instruments. For example, it happened that surgeons believed that they needed to wait a certain period of time after firing of the stapler when the staple formation was already completed, while others believe that they do not need to wait at all during the different operations of the stapler.

In view of this drawback of the state of the art, the object of the invention is to provide an indicator device which permits to better envision the tissue properties and compression time - dependent behavior after activation of the anvil jaw approximation mechanism.

This and other objects are achieved by an indicator device for indicating properties of body tissue subjected to surgical stapling as a function of tissue compression time according to claim 1.

The above object is also achieved by a demonstration device for training, explanation and sales activity according to claim 5.

The dependent claims cover advantageous embodiments of the invention.

According to the disclosure, the indicator device for indicating properties of body tissue subjected to surgical stapling as a function of tissue compression time comprises a housing and support structure, an actuating device received in the housing and having an adjustable compression time setting member and a display device supported by the housing and support structure and co-operating with the compression time setting member such that the display device visualizes a tissue property corresponding to a compression time set by the compression time setting member, wherein the display device is configured to visualize a reduced thickness feature of the clamped tissue corresponding to the set compression time.

This makes it possible to directly envisioning the relationship between the fluid evacuation state or thickness reduction of the body tissue and the set compression time or, in other words, the expired time period between anvil approximation (jaw closure) and "firing" of the stapler.

As a matter of fact, the lack of information about the behavior of the tissue during the several operations performed by means of surgical staplers was one of the reasons for the difficulties surgeons encountered by imagining the result of their intervention.

In accordance with a further aspect of the disclosure, a surgical stapling instrument (33) is provided which comprises:
- a housing having a body portion and a handle,
- a staple fastening assembly in the distal region of said instrument, the staple fastening assembly including a cartridge device which comprises at least one row of staples and defines an end surface, and an anvil which defines a staple forming surface which is movable relative to the cartridge device and adapted to cooperate with the cartridge device for forming the ends of the staples exiting from the cartridge device,
- a moving device adapted to move the anvil relative to the cartridge device,
- a staple driving device adapted to drive the staples out of the cartridge device towards the anvil,
wherein the stapling instrument (33) comprises an indicating device including:
- a timer received in the housing and configured to generate a timing signal which is indicative for a lapsed tissue compression time,
- an indicator supported by the housing and co-operating with the timer such that the indicator provides a visual or acoustic indication of a tissue compression status on the basis of said timing signal.

This enables the surgeon to have direct information regarding the tissue compression status and the optimal moment for "firing" the stapler.

These and other details and advantages of the present invention shall be made apparent from the accompanying drawings and the description thereof, which illustrate embodiments of the invention and, together with the general description of the invention given above, and the detailed description of the invention given below, serve to explain the principles of the present invention.

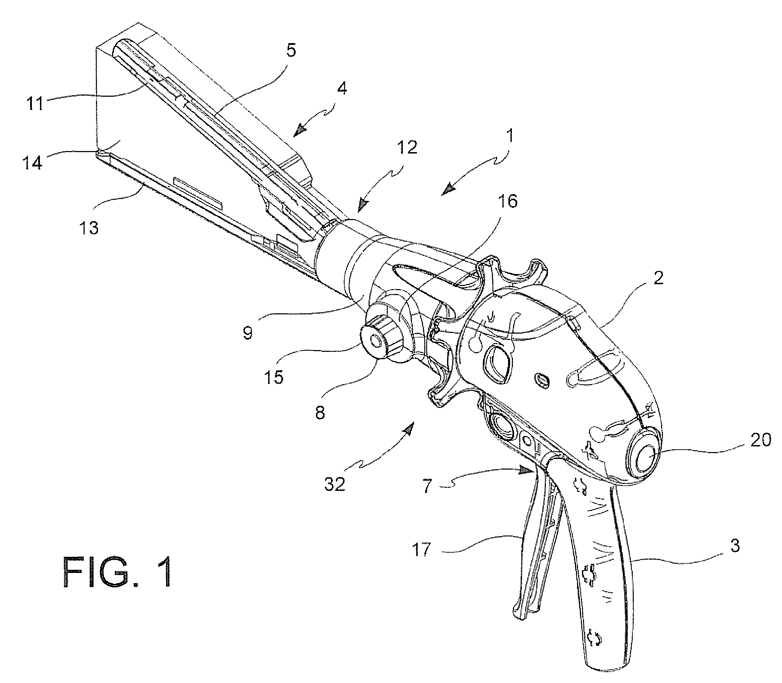

Fig. 1 illustrates an isometric view of a sales demo device incorporating an indicator device according to an embodiment of the invention;
Figs. 2 to 4 are side views of the device in figure 1 with an associated enlarged view of a compression time setting device thereof in different operational configurations, illustrating the functioning of the device in accordance with an embodiment;

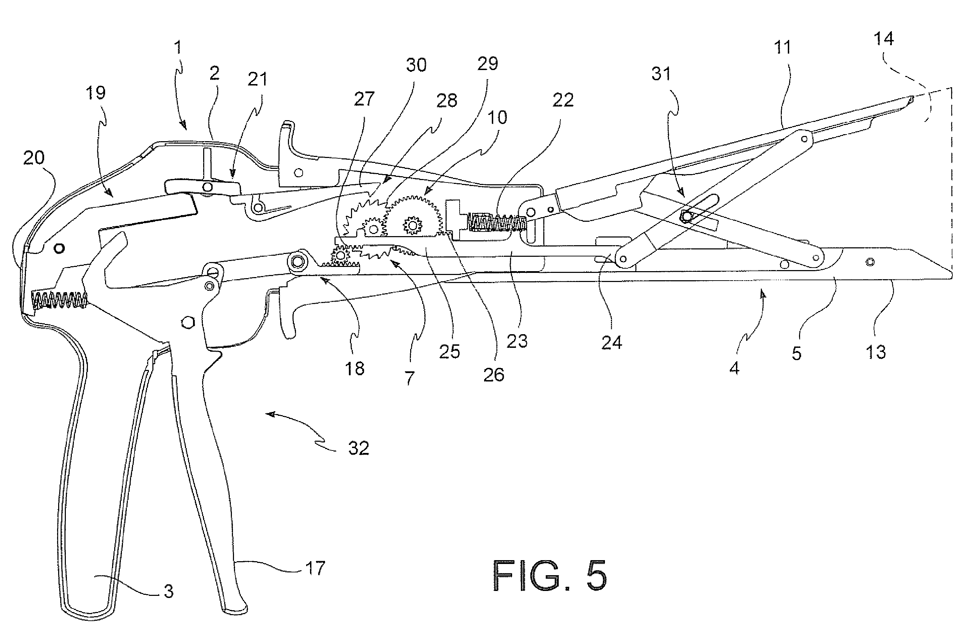

Fig. 5 illustrates a side view of the device in figure 1, wherein part of the housing is removed.

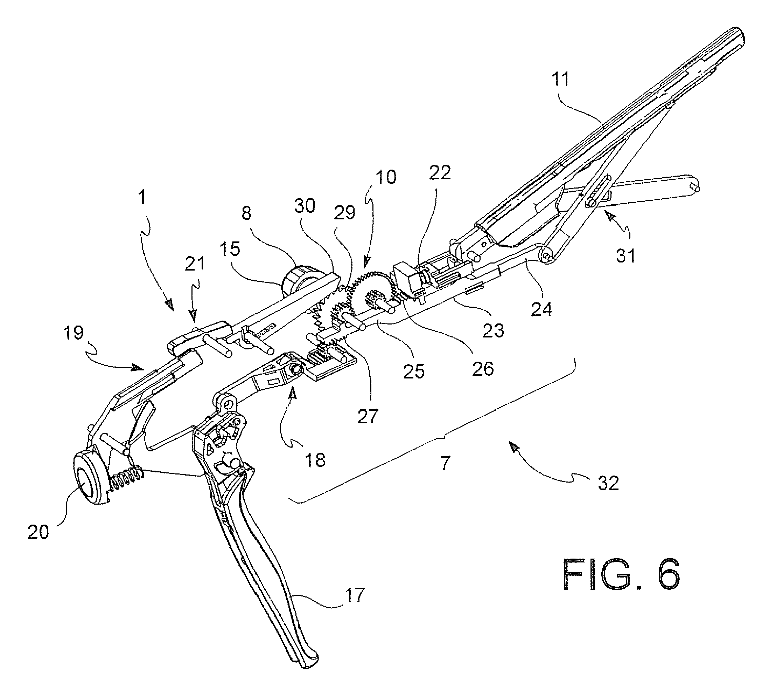

Fig. 6 illustrates an isometric view of the device in figure 1, wherein the housing and one jaw is removed.
Fig. 7 is a perspective view of a surgical stapling instrument having an indicator device in accordance with an embodiment of the invention.

Turning to the figures, the reference numeral 1 denotes an indicator device for indicating properties of body tissue subjected to surgical stapling as a function of tissue compression time.

The indicator device 1 comprises a pistol grip shaped housing 2 which forms a handle portion 3 and supports a display device 4 with at least one three-dimensional display member 5 or with a display screen 6 or window arranged at or in the housing 2 and visibly facing or protruding outward from the latter. The housing 2 further supports an actuating device 7 with an adjustable compression time setting member 8, for instance a manually operable rotary knob 15 arranged on a lateral side 9 of the housing 2 and a setting - transmission device 10 which cooperates with both the compression time setting member 8 and the display device 4.

The compression time setting member 8 allows the user to adjustably set or select a compression time (which throughout the present description is intended as the time period between the closure of the anvil and staple cartridge device jaws of a surgical stapler or endocutter and the firing of the stapler or endocutter, during which the tissue layers clamped between the stapler jaws are subjected to wide compression) and the display device 4 and the actuating device 7 are configured such that the display device 4 visualizes a reduced thickness feature of the clamped tissue corresponding to the compression time set by means of the compression time setting member 8.

In accordance with an embodiment, the display device 4 is configured to visualize the, possibly enlarged, distance between the stapler anvil and staple cartridge device or jaws corresponding to the set or selected tissue compression time.

In accordance with a further embodiment, the display device 4 is configured to visualize the possibly enlarged distance of the stapler anvil and staple cartridge device or jaws and/or the possibly enlarged shape and thickness reduction of the tissue clamped therebetween corresponding to the set or selected tissue compression time.

As already mentioned above, in accordance with an embodiment, the display device 4 is configured to visualize the reduced tissue thickness features in a magnified scale compared to the real size of a corresponding portion of tissue. This magnification of the visualized reduced tissue thickness feature with respect to the real tissue size allows the user to immediately see and understand the tissue behavior under wide compression without any need to get the eye particularly close to the three dimensional display member 5 or display screen 6.

It will be appreciated by those skilled in the art, that the display device 4 can comprise any suitable known visualization and screen display and control technology, as for instance a digital electronic display connected to an electronic control circuit which cooperates with the actuating device 7. An example for such a digital electronic display is a Liquid Crystal Display (LCD).

In accordance with an embodiment of the present invention, the three dimensional display member 5 comprises a distally extending staple cartridge device jaw dummy 11 rigidly connected to a distal end 12 of the housing 2 as well as a corresponding distally extending anvil jaw dummy 13 movably connected to the distal end 12 of the housing 2 so that it can be moved away from (open position) and approximated towards the cartridge device jaw dummy 11 (uncompressed or initial closed position) and further approximated to a compressed or final closed position, which will be explained in more detail below. Moreover, a clamped tissue dummy 14 comprising a reversibly deformable block of sponge or rubber or similar material, is connected between stapler jaw dummies 11 and 13.

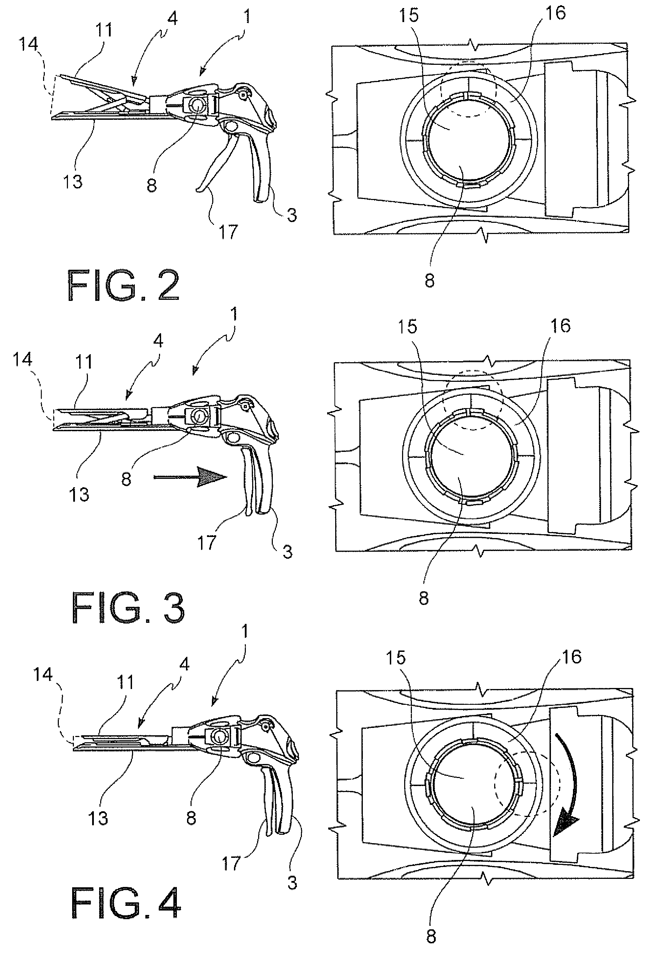

Apart from the above said compression time setting member 8 and setting-transmission device 10, the actuating device 7 also comprises a manual approximating member 17, e.g. an approximating lever or approximating knob arranged at the handle portion 3, and an approximating movement transmission and transformation mechanism 18 which is functionally connected to both the approximating member 17 and the stapler jaw dummies 11 and 13, so that, in response to an operation of approximating member 17, the stapler jaw dummies 11 and 13 and the tissue dummy 14 move or deform from the above said open position (fig. 2) to the initial closed position (fig. 3).

The stapler jaw dummies 11 and 13 and the tissue dummy 14 cooperate, by means of the setting-transmission mechanism 10, with the compression time setting member 8 such that, in response to the setting of a compression time by means of the compression time setting member 8, the stapler jaw dummies 11 and 13 and the tissue dummy 14 move or deform between the initial closed position (fig. 3) and the final closed position (fig. 4), thereby visualizing a magnified reduced thickness feature of the clamped tissue corresponding to the set tissue compression time.

In this context, it is to be noted that the expression "tissue compression time" can indicate a numeric value, such as 1, 2, ..., 15 seconds selectable by positioning rotary knob 15 such that a pointer-marker 16 formed on the rotary knob 15 is aligned with a corresponding marking of a time scale arranged around rotary knob 15 or, alternatively, the expression "tissue compression time" can indicate a succession of numeric or non-numeric intervals 16, preferably distinct by different colors, such as red (non sufficient tissue compression time) and green (sufficient tissue compression time) and possibly an initial marking indicating the beginning of tissue compression and an end marking indicating the completion of tissue compression, as required for optimal staple results.

In accordance with an embodiment (figs. 2, 3, 4), one or more intervals 16 with initial marking and end marking are formed along the adjustment path of the compression time setting member 8, and specifically around the rotary knob 15.

In accordance with an embodiment, the setting - transmission mechanism 10 comprises a rack and gear mechanism configured to transmit and convert the movement and positioning of the compression time setting member 8 in a movement and positioning of the stapler jaw dummies 11 and 13 between their initial closed position and final closed position, which in turn deform the tissue dummy 14 accordingly.

Analogously, also the approximating movement transmission mechanism 18 comprises a rack and gear mechanism configured to transmit and convert the activation movement of the manual approximating member 17 in an approximating movement of the stapler jaw dummies 11 and 13 from their open position to the initial closed position, which in turn deform the tissue dummy 14 accordingly.

The indicator device 1 further comprises release means 19 configured to release the manual approximating member 17 and approximating movement transmission mechanism 18 from their activated position (which corresponds to the closed position of the jaw dummies 11, 13) so that they can return to their rest position (which corresponds to the open position of the jaw dummies 11, 13).

In accordance with an embodiment, the release means 19 include a release button 20 arranged in housing 2 and a lock and release mechanism 21 configured to automatically lock the approximating movement transmission mechanism 18 in its activated position and to release both the approximating movement transmission mechanism 18 and the setting transmission mechanism 10 in response to an operation of release button 20, so that they can return to their rest positions.

The release mechanism 21 may comprise a ratchet 28 with a toothed wheel 29 connected with the setting - transmission gear 10 and a pawl 30 meshing with toothed wheel 29 so that it can move only in a direction corresponding to the closing direction of jaw dummies 11, 13. Pawl 30 is elastically preloaded in engagement with toothed wheel 29 and linked to the release button 20 by a lever mechanism which is configured to uncouple pawl 30 from toothed wheel 29 in response of an operation of release button 20.

Both the approximating movement transmission mechanism 18 and the setting transmission mechanism 10 are elastically biased towards their rest position, e.g. by a return spring 22 acting between housing 2 and a transmission rack 23 which has a distal end 24 linked to at least one jaw dummy 11, 13 (e.g. through a cross-link pantograph 31) and a proximal end 25 provided with a first toothed zone 26 meshing with the setting-transmission mechanism 10 and a second toothed zone 27 meshing with the approximation movement transmission mechanism 18.

In accordance with an embodiment of the invention, the indicator device 1 is embodied as or integrated in a demonstration device 32 for training, explanation and sales activity.

Those skilled in the art will immediately appreciate that the use in the indicator device 1 of a housing, actuating device and 3D display member which are of similar or identical shape of the housing, actuating device and tissue manipulation portion of a surgical stapling instrument allows a very realistic simulation of different tissue compression time intervals during training, explanation and sales activities.

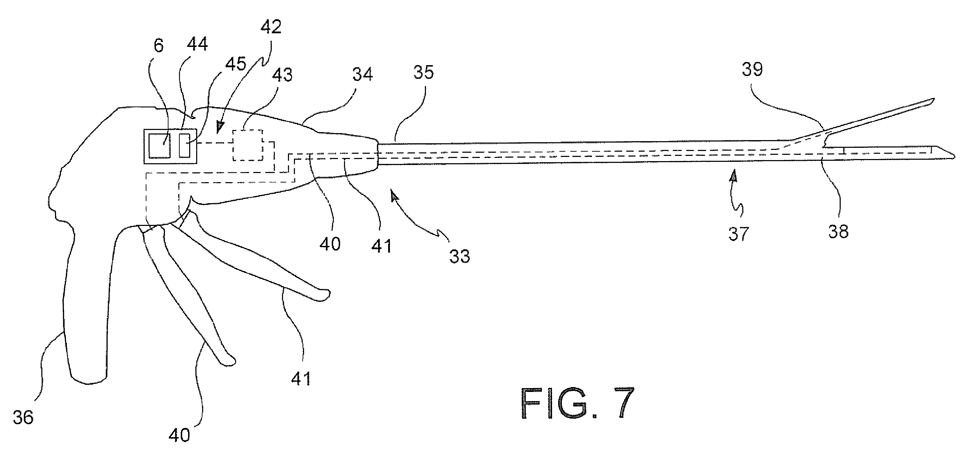

In accordance with a further aspect of the disclosure, a surgical stapling instrument 33 (Fig.7) is provided which comprises a housing 34 having a body portion 35 and a handle 36 as well as a staple fastening assembly 37 in the distal region of the instrument. The staple fastening assembly 37 includes a cartridge device 38 which comprises at least one row of staples and defines an end surface, and an anvil 39 which defines a staple forming surface which is movable relative to the cartridge device 38 and adapted to cooperate with the cartridge device 38 for forming the ends of the staples exiting from the cartridge device 38. The stapling instrument 33 comprises further a moving device 40 adapted to move the anvil 39 relative to the cartridge device 38 and a staple driving device 41 adapted to drive the staples out of the cartridge device 38 towards the anvil 39.

The stapling instrument 33 comprises an indicating device 42 having a timer 43 received in the housing 34 and configured to generate a timing signal which is indicative for a lapsed tissue compression time, as well as an indicator 44 supported by the housing 34 and co-operating with the timer 43 such that the indicator 44 provides a visual or acoustic indication of a tissue compression status on the basis of the timing signal.

In accordance with an embodiment, timer 43 is connected to the anvil moving device 40 and configured:
- to start a tissue compression time count when the anvil moving device 40 is completely activated and
- to generate a first timing signal on expiration of a preset optimum compression time period, e.g. 15 seconds, and/or
- to generate a second timing signal (which is different from the first timing signal) indicative of a yet insufficient tissue compression time, as long as the preset optimum compression time period, e.g. 15 seconds, has not yet expired.

This enables the surgeon to have direct information regarding the tissue compression status and the optimal moment for "firing" the stapler.

In accordance with embodiments, timer 43 can comprise a mechanical timing mechanism, e.g. a spring loaded clock work, or an electronic timing circuit.

The indicator 44 may include a display window with a mechanic indication pointer or an electronic display screen 6 as previously described in relation with embodiments of display device 4 and/or an acoustic sound emitter device 45.

While the present invention has been illustrated by description of several embodiments and while the illustrative embodiments have been described in considerable detail, it is not the intention to restrict or in any way limit the scope of the appended claims to such detail. Additional advantages and modifications may readily appear to those skilled in the art.

## Claims

1. An indicator device (1) for indicating properties of body tissue subjected to surgical stapling as a function of tissue compression time, said indicator device comprising a housing (2), an actuating device (7) received in the housing (2) and having an adjustable compression time setting member (8) and a display device (4) supported by the housing (2) and co-operating with the compression time setting member (8) such that, for a compression time value set by means of the compression time setting member (8), the display device (4) visualizes a reduced thickness feature of the clamped tissue corresponding to the set compression time value, wherein said display device (4) comprises a three dimensional display member (5) including a staple cartridge device jaw dummy (11) connected to the housing (2) and an anvil jaw dummy (13) connected to the housing (2) and movable with respect to the staple cartridge device jaw dummy (11) between an open position and an initial closed position and between said initial closed position and a final closed position, and a clamped tissue dummy (14) made of a reversibly deformable material connected between said stapler jaw dummies (11, 13),
wherein said actuating device (7) comprises a manual approximating member (17) arranged at a handle portion (3) of said housing (2) and an approximating movement transmission device (18) co-operating with said approximating member (17) and with said stapler jaw dummies (11, 13) such that, in response to an operation of said approximating member (17), said stapler jaw dummies (11, 13) move from said open position to said initial closed position,
wherein said actuating device (7) further comprises a setting-transmission device (10) co-operating with said compression time setting member (8) and with said stapler jaw dummies (11, 13) such that, in response to a tissue compression time setting movement of said compression time setting member (8), said stapler jaw dummies (11, 13) move between said initial closed position and said final closed position, thereby visualizing a magnified reduced thickness feature of the clamped tissue corresponding to the set tissue compression time value.

2. An indicator device (1) according to claim 1, wherein the display device (4) is configured to visualize a magnified distance between a stapler anvil and staple cartridge device corresponding to the set tissue compression time value.

3. An indicator device (1) according to claim 2, wherein the display device (4) is configured to visualize a magnified thickness reduction of a tissue clamped between a stapler anvil and staple cartridge device corresponding to the set tissue compression time value.

4. An indicator device (1) according to any one of the preceding claims, wherein said compression time setting member (8) comprises a rotary knob (15) having a pointer-marker which can be aligned with time markings of a time interval scale (16) arranged around said rotary knob (15), thereby setting said compression time value.

5. A demonstration device (32) for training, explanation and sales activity, comprising an indicator device (1) according to any one of the preceding claims.

## Patentansprüche

1. Anzeigevorrichtung (1) zum Anzeigen von Eigenschaften von Körpergewebe, das chirurgisch geklammert wird, als eine Funktion von Gewebekompressionszeit, wobei die Anzeigevorrichtung aufweist: ein Gehäuse (2), eine in dem Gehäuse (2) aufgenommene Betätigungsvorrichtung (7) mit einem Einstellbare-Kompressionszeit-Einstellelement (8) und eine Displayvorrichtung (4), die durch das Gehäuse (2) gestützt wird und mit dem Kompressionszeit-Einstellelement (8) so kooperiert, dass, für einen durch das Kompressionszeit-Einstellelement (8) eingestellten Kompressionszeitwert die Displayvorrichtung (4) eine Reduzierte-Dicke-Eigenschaft des geklammerten Gewebes korrespondierend zu dem eingestellten Kompressionszeitwert visualisiert,
wobei die Displayvorrichtung (4) aufweist: ein dreidimensionales Anzeigeelement (5) mit einem mit dem Gehäuse (2) verbundenen Klammermagazin-Klemmbacken-Dummy (11) und einem Gegenhalter-Klemmbacken-Dummy (13), das mit dem Gehäuse (2) verbunden und in Bezug auf das Klammermagazin-Klemmbacken-Dummy (11) bewegbar ist zwischen einer offenen Position und einer anfangs geschlossenen Position sowie zwischen der anfangs geschlossenen Position und einer abschließend geschlossenen Position, und ein Klammergewebe-Dummy (14) aus einem reversibel deformierbaren Material, das zwischen den Klammer-Klemmbacken-Dummys (11, 13) verbunden ist,
wobei die Betätigungsvorrichtung (7) ein manuelles Annäherungselement (17) umfasst, das auf einem Griffabschnitt (3) des Gehäuses (2) angeordnet ist, und eine Annäherungsbewegungsübertragungsvorrichtung (18), die mit dem Annäherungselement und den Klammer-Klemmbacken-Dummys (11, 13) so zusammenwirkt, dass, in Reaktion auf eine Betätigung des Annäherungselements (17), die Klammer-Klemmbacken-Dummys (11, 13) sich von der offenen Position zu der anfangs geschlossenen Position bewegen,
wobei die Betätigungsvorrichtung (7) weiterhin eine Einstellungs-Übertragungs-Vorrichtung (10) aufweist, die mit dem Kompressionszeit-Einstellelement (8) und den Klammer-Klemmbacken-Dummys (11, 13) so zusammenwirkt, dass, in Reaktion auf eine Gewebe-Kompressionszeit-Einstell-Bewegung des Kompressionszeit-Einstell-Elements (8), die Klammer-Klemmbacken-Dummys (11, 13) sich zwischen der anfangs geschlossenen Position und der abschließend geschlossenen Position bewegen und dabei eine vergrößerte Reduzierte-Dicke-Eigenschaft korrespondierend zu dem eingestellten Kompressionswert visualisieren.

2. Anzeigevorrichtung (1) nach Anspruch 1, wobei die Displayvorrichtung (4) konfiguriert ist zum Visualisieren einer vergrößerten Entfernung zwischen einem Klammer-Gegenhalter und einer Klammermagazinvoruchtung, die zu dem eingestellten Gewebekompressionszeitwert korrespondiert.

3. Anzeigevorrichtung (1) nach Anspruch 2, wobei die Displayvorrichtung (4) konfiguriert ist um eine vergrößerte Dicke-Reduktion eines Gewebes, das zwischen einem Klammer-Gegenhalter und einer Klammermagazinvorrichtung geklemmt ist, korrespondierend zu dem eingestellten Gewebe-Kompressionszeitwert zu visualisieren.

4. Anzeigevorrichtung (1) nach einem der vorherigen Ansprüche, wobei das Kompressionszeiteinstellelement (8) einen Drehknopf (15) mit einer Zeigermarkierung aufweist, die mit Zeit-Markierungen einer Zeit-Intervall-Skala (16), die um den Drehknopf (15) angeordnet ist, ausgerichtet werden kann, um somit den Kompressionszeitwert einzustellen.

5. Demonstrationsvorrichtung (32) für Training, Erklärung und Verkaufsaktivitäten, umfassend eine Anzeigevorrichtung (1) nach einem der vorherigen Ansprüche.

## Revendications

1. Dispositif indicateur (1) destiné à indiquer les propriétés d'un tissu corporel soumis à un agrafage chirurgical en fonction d'un temps de compression du tissu, ledit dispositif indicateur comprenant un logement (2), un dispositif d'actionnement (7) reçu dans le logement (2) et ayant un élément de réglage du temps de compression ajustable (8) et un dispositif d'affichage (4) supporté par le logement (2) et coopérant avec l'élément de réglage du temps de compression (8) de telle sorte que, pour une valeur du temps de compression fixée au moyen de l'élément de réglage du temps de compression (8), le dispositif d'affichage (4) visualise une caractéristique d'épaisseur réduite du tissu maintenu correspondant à la valeur du temps de compression fixée,
dans lequel ledit dispositif d'affichage (4) comprend un élément d'affichage tridimensionnel (5) incluant une mâchoire factice (11) du dispositif de cartouche d'agrafes reliée au logement (2) et une mâchoire factice formant une enclume (13) reliée au logement (2) et mobile par rapport à la mâchoire factice (11) du dispositif de cartouche d'agrafes entre une position ouverte et une position fermée initiale et entre ladite position fermée initiale et une position fermée finale, et un tissu factice maintenu (14) constitué d'un matériau déformable de façon réversible relié entre lesdites mâchoires factices (11, 13) de l'agrafeuse,
dans lequel ledit dispositif d'actionnement (7) comprend un élément de rapprochement manuel (17) disposé sur une partie de poignée (3) dudit logement (2) et un dispositif de transmission du mouvement de rapprochement (18) coopérant avec ledit élément de rapprochement (17) et avec lesdites mâchoires factices de l'agrafeuse (11, 13), de sorte qu'en réponse à un fonctionnement dudit élément de rapprochement (17), lesdites mâchoires factices de l'agrafeuse (11, 13) se déplacent de ladite position ouverte à ladite position initiale fermée,
dans lequel ledit dispositif d'actionnement (7) comprend en outre un dispositif de transmission de réglage (10) coopérant avec ledit élément de réglage du temps de compression (8) et avec lesdites mâchoires factices de l'agrafeuse (11, 13) de sorte que, en réponse à un mouvement de réglage du temps de compression du tissu dudit élément de réglage du temps de compression (8), lesdites mâchoires factices de l'agrafeuse (11, 13) se déplacent entre ladite position fermée initiale et ladite position fermée finale, ce qui permet de visualiser une caractéristique d'épaisseur réduite agrandie du tissu maintenu correspondant à la valeur du temps de compression fixée du tissu.

2. Dispositif indicateur (1) selon la revendication 1, dans lequel le dispositif d'affichage (4) est configuré pour visualiser une distance agrandie entre une enclume de l'agrafeuse et un dispositif de cartouche d'agrafes correspondant à la valeur du temps de compression fixée d'un tissu.

3. Dispositif indicateur (1) selon la revendication 2, dans lequel le dispositif d'affichage (4) est configuré pour visualiser une réduction d'épaisseur agrandie d'un tissu maintenu entre une enclume d'agrafeuse et un dispositif de cartouche d'agrafes correspondant à la valeur du temps de compression fixée du tissu.

4. Dispositif indicateur (1) selon l'une quelconque des revendications précédentes, dans lequel ledit élément de réglage du temps de compression (8) comprend un bouton tournant (15) ayant un marqueur-pointeur qui peut être aligné avec des graduations de temps d'une échelle d'intervalle temporel (16) disposées autour dudit bouton tournant (15) permettant de régler ladite valeur du temps de compression.

5. Dispositif de démonstration (32) pour une activité de formation, d'explication et de ventes, comprenant un dispositif indicateur (1) selon l'une quelconque des revendications précédentes.
